# EUROPÄISCHE PATENTSCHRIFT

(11) **EP 1 115 715 B1**
(45) Veröffentlichungstag und Bekanntmachung des Hinweises auf die Patenterteilung: **15.12.2004**
(21) Anmeldenummer: 99947373.9
(22) Anmeldetag: 16.09.1999
(51) Int. Cl.: C07D 307/08

(54) **VERFAHREN ZUR HERSTELLUNG VON TETRAHYDROFURAN**
METHOD FOR PRODUCING TETRAHYDROFURAN
PROCEDE DE PRODUCTION DE TETRAHYDROFURANE

(30) Priorität: 18.09.1998 DE 19842847
(43) Veröffentlichungstag der Anmeldung: 18.07.2001
(73) Patentinhaber: BASF AKTIENGESELLSCHAFT, 67056 Ludwigshafen (DE)
(72) Erfinder: FISCHER, Rolf, D-69121 Heidelberg (DE); LIANG, Shelue, D-67071 Ludwigshafen (DE); PINKOS, Rolf, D-67098 Bad Dürkheim (DE)
(74) Vertreter: Isenbruck, Günter
(86) Internationale Anmeldenummer: PCT/EP1999/006876
(87) Internationale Veröffentlichungsnummer: WO 2000/017181

(56) Entgegenhaltungen:
- EP-A- 0 443 392
- WO-A-97/26255
- DE-A- 2 461 922

## Beschreibung

Die Erfindung betrifft ein Verfahren zur Herstellung von Tetrahydrofuran (THF) durch Umsetzung einer 1,4-Butandiol enthaltenden Reaktionsmischung an einem sauren Katalysator.

Verfahren zur Herstellung von THF aus 1,4-Butandiol sind seit langem bekannt. In K. Weissermel, H.-J. Arpe, Industrielle Organische Chemie, VCH Verlagsgesellschaft, D 69451 Weinheim, 1994, Seite 111, ist die Umsetzung von 1,4-Butandiol zu THF durch Wasserabspaltung unter Zugabe von Phosphorsäure, Schwefelsäure oder sauren Ionenaustauschern beschrieben. Dabei wird das mit Säure versetzte 1,4-Butandiol erhitzt und in dem Maß durch 1,4-Butandiol ergänzt, wie THF/Wasser herausdestilliert.

Nachteilig an diesem Verfahren zur Herstellung von THF ist, daß das üblicherweise eingesetzte 1,4-Butandiol vor dem Einsatz zunächst gereinigt werden muß. Die Reinigung erfolgt meist durch aufwendige mehrstufige Destillation, wobei unerwünschte leicht- und/oder schwersiedende Bestandteile, einschließlich Wasser, abgetrennt werden. Anschließend wird dieses wasserfreie Rein-Butandiol zu THF umgesetzt, wobei Wasser und unerwünschte Nebenprodukte entstehen. Daher muß das als Produkt erhaltene THF nach der Umsetzung wiederum mehrstufig destillativ gereinigt werden. Es müssen also zweimal vergleichbare, aufwendige Reinigungsund Abtrennungsschritte durchgeführt werden.

In der DE-A-196 01 375 wird dieses Problem so gelöst, daß zunächst die in einer wäßrigen Butandiollösung enthaltenen leichtflüchtigen organischen Verbindungen destillativ entfernt werden. Die so vorgereinigte wäßrige Bütandiollösung wird an einem sauren Aluminiumoxidkatalysator dehydratisiert und die gewonnene THFreiche Fraktion zur Gewinnung von reinem THF destilliert. Dabei wird durch die Anwesenheit von Wasser in der Butandiollösung die Selektivität des Katalysators erhöht. Üblicherweise fallen solche als Ausgangsstoff eingesetzten wäßrigen Butandiollösungen in der Technik bei der Herstellung von Butandiol aus Acetylen und Formaldehyd und anschließender Hydrierung des entstandenen Butindiols an. Diese Hydrierausträge enthalten neben Wasser und Butandiol Leichtsieder wie Methanol, Propanol und Butanol.

In den beiden prioritätsälteren, nicht-vorveröffentlichten WO 99/35136 und WO 99/35113 ist auch schon vorgeschlagen worden, THF zusammen mit γ-Butyrolacton und gegebenenfalls zusätzlich mit 1,4-Butandiol in Mehrstufenprozessen herzustellen, bei denen ausgehend von z.B. Maleinsäuredimethylester in zwei oder drei Teilstofen an unterschiedlichen Katalysatoren hydriert und cyclisiert wird. Eine typische Zusammensetzung enthält nach diesen Teilstufen 53 % γ-Butyrolacton, 34 % THF, 7 % 1,4-Butandiol und 3 % Nebenprodukte bei einer Gesamtumwandlungsrate von 97 %. Eine Entfernung von aus der THF-Synthese stammendem Wasser wird nur im Zusammenhang mit dem Rezyklieren der Gasanteile erwähnt. Die Flüssiganteile des Reaktionsauslasses sind (siehe Fig. 1 beider WO's) nach destillativer Anftrennung THF, Methanol, γ-Butyrolacton, eine rezyklierbare Fraktion aus 1,4-Butandiol und dem Edukt Maleinsäuredimethylester, schwersiedende organische Nebenprodukte und eine Fraktion aus Wasser und niedrigsiedenden organischen Nebenprodukten. Es wird ausdrücklich erwähnt, daß diese speziellen mehrstufigen Verfahren einem Verfahren mit 1,4-Butandiol als Edukt vorzuziehen seien.

DE-A-24 619 222 betrifft ein Verfahren zur Herstellung von Tetrahydrofuran aus 1,4-Butandiol, worin 1,4-Butandiol in der Dampfphase in Anwesenheit eines Dehydratisierungskatalysators aus der Gruppe Zeolithen, Siliciumoxid, Aluminiumoxid, Siliciumoxid-Aluminiumoxid, Silicium-Magnesiumoxid und sauren Tonerden umgesetzt wird. Als 1,4-Butandiol enthaltendes Reaktionsgemisch kann eine Mischung aus 1,4-Butandiol, 1,2-Butandiol und 2-Methyl-1,3-propandiol eingesetzt werden, daß durch Umsetzung von Propylen, Sauerstoff und einer Carbonsäure, anschließende Hydroformylierung darauffolgende Hydrierung und anschließende Entesterung hergestellt wird.

EP-A-0 443 392 betrifft ein Verfahren zur Herstellung von THF oder von THF und γ-Butyrolacton aus dem Hydrierprodukt der Hydrierung von Maleinsäure, Bernsteinsäure, Maleinsäureanhydrid, Bernsteinsärueanhydrid und/oder fumarsere Anhydrid, wobei das rohe Hydrierprodukt ohne vorherige Aufarbeitung mit Protonsäuren behandelt wird und aus der erhaltenen Reaktionsmischung reines Tetrahydrofuran oder γ-Butyrolacton destillativ isoliert wird. Gemäß der Beispiele enthalten die eingesetzten Reaktionsmischungen über 26 Gew.-% Wasser.

Von besonderem Interesse ist die effiziente Herstellung von THF aus im wesentlichen nicht-wäßrigen Butandiollösungen, die man z.B. durch Hydrierung von Maleinsäure oder deren Derivaten, ohne vorherige Abtrennung der in dem

Hydrieraustrag enthaltenen Leichtsieder wie Methanol, Propanol etc. und anderer Nebenprodukte, erhält. Ein Problem dabei ist, daß bei den Reaktionsbedingungen der Cyclisierung von 1,4-Butandiol zu THF (intramolekulare Veretherung) auch die anderen in der Reaktionsmischung enthaltenen Alkohole verethern könnten (intermolekulare Veretherung). Weiterhin ist die Bildung von Olefinen aus Alkoholen, z.B. von Buten aus Butanol möglich. Diese Nebenprodukte würden sowohl die Ausbeute an THF als auch die Standzeiten des Katalysators verringern.

Die Aufgabe der vorliegenden Erfindung ist es daher, ein Verfahren zur Herstellung von THF aus weitgehend wasserfreiem 1,4-Butandiol bereitzustellen, bei dem die eingesetzte, Butandiol enthaltende Reaktionsmischung nicht vorgereinigt werden muß, und die Bildung nennenswerter Mengen von Nebenprodukten vermieden wird.

Diese Aufgabe wird gelöst durch ein Verfahren zur Herstellung von THF durch Umsetzung einer 1,4-Butandiol enthaltenden Reaktionsmischung aus der Hydrierung von Maleinsäurediestern an einem sauren Katalyastor, wobei die Reaktionsmischung weitere von 1,4-Butandiol verschiedene Alkohole enthält und einen Wassergehalt unter 5 Gew.-% aufweist.

Bevorzugt liegt der Wassergehalt unter 2 Gew.-%, besonders bevorzugt unter 1 Gew.-% zu.

Das erfindungsgemäße Verfahren hat den Vorteil, daß die eingesetzten 1,4-Butandiol enthaltenden Reaktonsmischungen nicht vor der Umsetzung zu THF an einem sauren Katalysator vorgereinigt werden müssen. 1,4-Butandiol wird in Gegenwart von weiteren Alkoholen zu THF cyclisiert, ohne daß nennenswerte Mengen Nebenprodukte entstehen. Damit entfällt die aufwendige Vorreinigung, so daß Kosten eingespart werden können.

Die Umsetzung von 1,4-Butandiol, und zwar bevorzugt als Hauptkomponente, insbesondere zu mehr als 50 Gew.-%, enthaltenden Reaktionsmischungen zu THF kann bevorzugt in der Flüssigphase oder auch in der Gasphase durchgeführt werden.

Die Hydrierung kann in bekannter Weise in der Gas- oder Flüssigphase durchgeführt werden. Beispielsweise kann Maleinsäuredimethylester durch Hydrierung an einem Katalysator, z.B. Kupferchromit, unter erhöhtem Druck und erhöhter Temperatur in der Gasphase umgesetzt werden. Der gewonnene Hydrieraustrag, der als Zulauf in der erfindungsgemäßen Umsetzung eingesetzt wird, enthält im allgemeinen 5 - 85 Gew.-% Butandiol und 15 - 95 Gew.-% Alkohol, bevorzugt 10 bis 70 Gew.-% Butandiol und 15 bis 70 Gew.-% Alkohol, besonders bevorzugt 15 bis 60 Gew.-% Butandiol und 15 bis 50 Gew.-% Alkohol. Daneben können Produkte wie γ-Butyrolacton oder Bernsteinsäurediester im Bereich bis z.B. 30 Gew.-%, enthalten sein. Die Gehalte an γ-Butyrolacton oder Bernsteinsäurediester sind im allgemeinen für das Verfahren unkritisch, bevorzugt liegen sie aber bei nicht mehr als dem halben Gewichtsanteil des Butandiolanteils.

Weiterhin kann Wasser in einem Gehalt von im allgemeinen unter 5 Gew.-%, bevorzugt unter 2 Gew.-%, besonders bevorzugt unter 1 Gew.-% vorhanden sein, sowie geringe Mengen weiterer Verbindungen. Es ist möglich, daß im Hydrieraustrag bereits THF vorliegt, wobei der THF-Gehalt für das Verfahren nicht kritisch ist und z.B. zwischen 10 und 30 Gew.-% liegen kann. In einer bevorzugten Ausführungsform enthält der Zulauf des erfindungsgemäßen Verfahrens 15 bis 60 Gew.-% Butandiol, 15 bis 50 Gew.-% Alkohol, bis zu 30 Gew.-% γ-Butyrolacton und/oder Bernsteinsäurediester, wobei dieser Anteil maximal die Hälfte des Butandiol-Anteils beträgt, und bis zu 30 Gew.-% THF, wobei weniger als 2 Gew.-% Wasser, bezogen auf die Summe aller anderen Komponenten als 100 Gew.-%, enthalten sind.

Anstelle des gesamten Hydrieraustrags kann auch nur ein Teilstrom des Hydrieraustrags der Umsetzung zu THF zugeführt werden. Das Reaktionsprodukt der Umsetzung zu THF kann denselben Aufarbeitungskolonnen zugeführt werden wie der nicht weiter umgesetzte Teilstrom des Hydrieraustrags, da beide ähnliche Verunreinigungen und Nebenprodukte aufweisen. So brauchen nicht für vergleichbare Trennaufgaben unterschiedliche Apparaturen betrieben zu werden.

Als Alkohole sind in dem erfindungsgemäßen Verfahren zur Herstellung von THF im allgemeinen aliphatische Alkohole, die bevorzugt einwertig sind, enthalten. Besonders bevorzugt sind einwertige aliphatische Alkohole mit 1 bis 7 Kohlenstoffatomen, davon sind Methanol, Ethanol, iso- und n- Propanol und n-Butanol ganz besonders bevorzugt.

Die Umsetzung erfolgt im allgemeinen bei 50 bis 300°C, bevorzugt bei 65 bis 270°C, besonders bevorzugt bei 65 bis 240°C. Sie wird in einem Druckbereich von im allgemeinen 0,5 bis 80 bar, bevorzugt 0,8 bis 60 bar, besonders bevorzugt 1 bis 40 bar durchgerührt.

In Abhängigkeit von den gewählten Temperatur- und Druckbedingungen können das gebildete THF und Wasser sowie der Alkohol von der flüssigen in die gasförmige Phase übergehen oder in der flüssigen Phase verbleiben. Bei niedrigen Reaktionsdrucken wird der Produktstrom den Reaktor gasförmig verlassen.

Bei einer bevorzugten Variante erfolgt die Umsetzung zu THF im Reaktor der Hydrierung entweder in der Gas- oder insbesondere der Flüssigphase in Gegenwart von Wasserstoff.

Als Katalysatoren können prinzipiell alle sauer wirkenden Stoffe, in homogen löslicher oder heterogener Form, eingesetzt werden. Beispiele für homogen lösliche Katalysatoren sind Phosphorsäure und Schwefelsäure.

Bevorzugt werden heterogene, feste saure Katalysatoren eingesetzt. Beispiele dafür sind die festen Oxide der Gruppen 3 bis 15 des Periodensystems der Elemente, wobei ein Katalysator mehrere Elemente der vorgenannten Gruppen enthalten kann, saure Ionenaustauscher und Heteropolysäuren. Bevorzugt werden feste Oxide, die mindestens ein Element der Gruppen 4, 6, 13 und 14 des Periodensystems der Elemente enthalten, besonders bevorzugt TiO₂, ZrO₂, CrO₂, γ-Al₂O₃, SiO₂ und/oder SnO₂ eingesetzt, wobei die Oxide auch untereinander vergesellschaftet, wie in Zeolithen, vorliegen können. Des weiteren können saure Tonerden, Schichtsilikate und Montmorillonite als feste saure Katalysatoren eingesetzt werden. Ganz besonders bevorzugt sind Katalysatoren, die mindestens eine Verbindung ausgewählt aus γ-Al₂O₃ und TiO₂ enthalten. Die Katalysatoren können zur Erhöhung ihrer Acidität mit Säuren wie Phosphorsäure oder Schwefelsäure vorbehandelt werden.

Die Umsetzung kann an suspendierten Katalysatoren in der Flüssigphase oder an fest angeordneten Katalysatoren in der Gasphase durchgeführt werden. Beispielsweise kann die Umsetzung von 1,4-Butandiol zu THF in der Gasphase in einem Wirbelbettreaktor oder einem Rohreaktor erfolgen. Der Rohrreaktor kann in der Riesel- oder Sumpffahrweise betrieben werden. Wird der Rohrreaktor in der Sumpffahrweise betrieben, können sich die in der Reaktionsmischung enthaltenen Hochsieder aus der Hydrierung von Maleinsäurederivaten im Reaktorsumpf ansammeln und als Teilstrom ausgeschleust werden. Dieser kann verworfen werden oder in frühere Verfahrensstufen, z.B. die Hydrierung, zurückgeführt werden.

Die Zulaufmenge beträgt pro Liter Katalysator im allgemeinen 0,1 bis 10 kg/h, bevorzugt 0,3 bis 5 kg/h, besonders bevorzugt 0,5 bis 3 kg/h.

Der Umsatz des in der Reaktionsmischung vorhandenen 1,4-Butandiols zu THF beträgt im allgemeinen 99 bis 100 %. Somit entspricht der Austrag nach der Umsetzung von 1,4-Butandiol zu THF (Cyclisierungsaustrag) im wesentlichen seiner Zulaufzusammensetzung, mit dem Unterschied, daß das im Zulauf enthaltene 1,4-Butandiol zu THF und Wasser umgesetzt wurde. Der Cyclisierungsaustrag enthält im allgemeinen THF, Reaktionswasser und Alkohol. Daneben können z.B. γ-Butyrolacton und Bernsteinsäureester enthalten sein. Sie werden bei der Reaktion nicht beeinflußt.

Anhand der Zusammensetzung des Cyclisierungsaustrags wird deutlich, daß bei der Umsetzung von 1,4-Butandiol enthaltenden Reaktionsmischungen zu THF an sauren Katalysatoren in Gegenwart weiterer Alkohole keine nennenswerte Bildung von Nebenprodukten, wie von Ethern durch intermolekulare Reaktion oder Olefinen, auftritt.

Der Cyclisierungsaustrag kann mit dem Fachmann bekannten Methoden destillativ aufgearbeitet werden. EP-B 0 485 484 beschreibt verschiedene Verfahren zur Gewinnung von THF aus Mischungen, die THF, einen oder mehrere niedrigsiedende Alkohole und Wasser enthalten. So kann die Gewinnung beispielsweise durch extraktive Destillation unter Zugabe einer weiteren Komponente wie 1,4-Butandiol, Wasser, Ethylenglykol und anderen erfolgen. EP-B 0 485 484 beschreibt weiterhin ein Verfahren zur Gewinnung von THF aus den erwähnten Mischungen, das zwei aufeinanderfolgende Destillationen, wobei die erste bei niedrigerem Druck als die zweite durchgeführt wird, und eine zwischen den Destillationen durchgeführte Kondensation umfaßt. Das aus der zweiten Destillation gewonnene, an Nebenprodukten angereicherte Gemisch wird mit dem Strom der ersten Destillation wiederum kondensiert, und das in der zweiten Destillation gewonnene reine THF wird abgetrennt.

Besonders vorteilhaft ist, daß der vorhandene Alkohol wieder in eine vorhergehende Stufe, z.B. Veresterung von Maleinsäureanhydrid, zurückgeführt werden kann.

In einer bevorzugten Ausführungsform wird die erfindungsgemäße Umsetzung in einem Rohrreaktor durchgeführt. Als saure Katalysatoren werden γ-Al₂O₃ und/oder SiO₂ und/oder TiO₂, bevorzugt γ-Al₂O₃ eingesetzt. Es wird, sofern der Eduktstrom nicht bereits die erforderliche Temperatur aufweist, mittels einer Heizung aufgeheizt, so daß die Temperatur in der Katalysatorzone 50 bis 300°C, bevorzugt 65 bis 270°C, besonders bevorzugt 65 bis 240°C beträgt. Anschließend wird die 1,4-Butandiol enthaltende Reaktionsmischung (Zulauf), vorzugsweise der Hydrieraustrag aus der Hydrierung von Maleinsäurediestern, über den Sumpf des Reaktors zugefahren. Der Zulauf beträgt pro Liter Katalysator im allgemeinen zwischen 100 und 10000 ml/h, bevorzugt zwischen 200 und 2400 ml/h, besonders bevorzugt zwischen 500 und 2000 ml/h. Nach der Einstellung eines stationären Zustandes beträgt die Temperatur am Anfang der Katalysatorzone 50 bis 150°C, bevorzugt 65 bis 100°C, besonders bevorzugt ca. 70°C und in der Mitte 150 bis 300°C, bevorzugt 150 bis 250°C, besonders bevorzugt ca. 200°C. Je nach Temperatur- und Druckbedingungen verdampft der Produktstrom oder liegt in der Flüssigphase vor. Bei Temperaturen von 70°C am Anfang der Katalysatorzone und 200°C in der Mitte und Normaldruck verdampft der Produktstrom, so daß ein gasförmiger Reaktionsaustrag erhalten wird.

THF ist ein wichtiges Lösungsmittel für viele Hochpolymere und dient des weiteren zur Herstellung von Polytetramethylenglykol, das ein Zwischenprodukt bei der Herstellung von Polyurethanen und Spandexfasern ist.

Das nachfolgende Beispiel erläutert die Erfindung zusätzlich.

### Beispiel

Die Prozentangaben sind durch Gaschromatographie ermittelte Gewichtsprozente (Methode mit innerem Standard).

### Herstellung einer 1,4-Butandiol enthaltenden Reaktionsmischung (Zulauf)

Der Zulauf wurde durch Hydrierung von Maleinsäuredimethylester an einem Kupferchromit-Katalyastor in der Gasphase bei 60 bar und Temperaturen von 180 bis 190°C gewonnen. Er hatte die folgende Zusammensetzung (Gew.-%): 40,1% Methanol, 6,5 % THF, 0,6 % n-Butanol, 8,4 % γ-Butyrolacton, 37,9 % 1,4-Butandiol, 0,1 % 4-Hydroxybuttersäuremethylester, 2 % Bernsteinsäuredimethylester, 0,7 % 2-(4-Hydroxybutoxy)tetrahydrofuran sowie Wasser und mehrere weitere Verbindungen, deren Gehalte unter 0,1 % lagen.

### Cyclisierung zu THF

In einen Rohrreaktor wurden 100 ml (ca. 63 g) saures γ-Al₂O₃ gefüllt (4 mm Stränge). Danach wurde mittels einer außenliegenden Heizung so aufgeheizt, daß die Temperatur in der mittleren Katalysatorzone 200°C betrug. Dann wurde der Zulauf (Zusammensetzung siehe oben) über den Sumpf des Reaktors zugefahren (50 ml/h). Nach 30 h stellte sich ein stationärer Zustand ein. Dabei betrug die Temperatur am Anfang der Katalysatorzone ca. 70°C, in der Mitte ca. 200°C. Am Anfang der Katalysatorzone verdampfte praktisch der gesamte Produktstrom, so daß nur ca. 5 % der Katalysatorzone mit Flüssigkeit benetzt waren. Im Reaktionsaustrag fanden sich 0,1 % Dimethylether, 39 % Methanol, 37,7 % THF, 0,5 % n-Butanol, 8,4 % γ-Butyrolacton, 0,2 % 1,4-Butandiol, 2,2 % Bernsteinsäuredimethylester sowie einige weitere Produkte, deren Gehalte unter 0,1 % lagen und Wasser. Die einzelnen Gehalte der angegebenen Produkte schwankten leicht von Probe zu Probe, veränderten sich aber nicht wesentlich. Auch nicht, als der Zulauf auf 80, 120 und 240 ml/h gesteigert wurde. Insgesamt wurden 8 kg Feed zugefahren. Der Katalyastor zeigte auch am Ende der Versuchsfahrt keine Desaktivierungsanzeichen. Die Katalysatorstränge waren unverändert.

## Patentansprüche

1. Verfahren zur Herstellung von THF durch Umsetzung einer 1,4-Butandiol enthaltenden Reaktionsmischung aus der Hydrierung von Maleinsäurediestern an einem sauren Katalyastor, **dadurch gekennzeichnet, daß** die Reaktionsmischung weitere von 1,4-Butandiol verschiedene Alkohole enthält und einen Wassergehalt unter 5 Gew.-% aufweist.

2. Verfahren nach Anspruch 1, **dadurch gekennzeichnet, dass** der Wassergehalt unter 2 Gew.-% liegt.

3. Verfahren nach Anspruch 1 oder 2, **dadurch gekennzeichnet, daß** als Alkohol ein einwertiger, aliphatischer C₁₋₇-Alkohol enthalten ist.

4. Verfahren nach Anspruch 3, **dadurch gekennzeichnet, daß** als Alkohol Methanol, Ethanol, Propanol oder n-Butanol enthalten ist.

5. Verfahren nach einem der Ansprüche 1 bis 4, **dadurch gekennzeichnet, daß** die Katalysatoren mindestens ein Element der 4., 6., 13. oder 14. Gruppe des Periodensystems der Elemente enthalten.

6. Verfahren nach Anspruch 5, **dadurch gekennzeichnet, daß** die Katalysatoren mindestens eine Verbindung ausgewählt aus γ-Al₂O₃ oder TiO₂ enthalten.

7. Verfahren nach einem der Ansprüche 1 bis 6, **dadurch gekennzeichnet, daß** die Umsetzung an suspendierten Katalysatoren in der Flüssigphase oder an fest angeordneten Katalysatoren in der Gasphase durchgeführt wird.

8. Verfahren nach einem der Ansprüche 1 bis 7, **dadurch gekennzeichnet, daß** die Reaktionsmischung 15 bis 60 Gew.-% 1,4-Butandiol, 15 bis 50 Gew.-% weitere(n) Alkohole, bis zu 30 Gew.-% γ-Butyrolacton und/oder Bernsteinsäurediester, wobei dieser Anteil maximal die Hälfte des Butandiol-Anteils beträgt, und bis zu 30 Gew.-% THF enthält, und wobei weniger als 2 Gew.-% Wasser, bezogen auf die Summe aller anderen Komponenten als 100 Gew.-%, enthalten sind.

9. Verfahren nach einem der Ansprüche 1 bis 8, **dadurch gekennzeichnet, daß** die Umsetzung in der Gasphase durchgeführt wird.

10. Verfahren nach einem der Ansprüche 1 bis 9, **dadurch gekennzeichnet, daß** die Umsetzung bei einer Temperatur von 50 bis 300°C und in einem Druckbereich von 0,5 bis 80 bar durchgeführt wird.

## Claims

1. A process for preparing THF by reaction of a 1,4-butanediol-containing reaction mixture from the hydrogenation of maleic diesters over an acid catalyst, wherein the reaction mixture further comprises additional alcohols other than 1,4-butanediol and has a water content of less than 5% by weight.

2. The process according to claim 1, wherein the water content is less than 2% by weight.

3. The process according to claim 1 or 2, wherein a monohydric, aliphatic C₁₋₇-alcohol is present as alcohol.

4. The process according to claim 3, wherein methanol, ethanol, propanol or n-butanol is present as alcohol.

5. The process according to any of claims 1 to 4, wherein the catalysts comprise at least one element of group 4, 6, 13 or 14 of the Periodic Table of the Elements.

6. The process according to claim 5, wherein the catalysts comprise at least one compound selected from among γ-Al₂O₃ and TiO₂.

7. The process according to any of claims 1 to 6, wherein the reaction is carried out in the liquid phase over suspended catalysts or in the gas phase over fixed-bed catalysts.

8. The process according to any of claims 1 to 7, wherein the reaction mixture comprises from 15 to 60% by weight of 1,4-butanediol, from 15 to 50% by weight of further alcohols, up to 30% by weight of γ-butyrolactone and/or succinic diesters, with this proportion being not more than half the proportion of butanediol, and up to 30% by weight of THF, with less than 2% by weight of water, based on the sum of all other components as 100%, being present.

9. The process according to any of claims 1 to 8, wherein the reaction is carried out in the gas phase.

10. The process according to any of claims 1 to 9, wherein the reaction is carried out at a temperature of from 50 to 300°C and in a pressure range from 0.5 to 80 bar.

## Revendications

1. Procédé de préparation de THF au moyen de la réaction d'un mélange réactionnel, contenant du 1,4-butanediol issu de l'hydrogénation de diesters d'acide maléique, sur un catalyseur acide, **caractérisé en ce que** le mélange réactionnel contient d'autres alcools différents du 1,4-butanediol et qu'il présente une teneur en eau inférieure à 5% en poids.

2. Procédé selon la revendication 1, **caractérisé en ce que** la teneur en eau est inférieure à 2% en poids.

3. Procédé selon la revendication 1 ou 2, **caractérisé en ce qu'**un monoalcool aliphatique en C₁ à C₇ est contenu en tant qu'alcool.

4. Procédé selon la revendication 3, **caractérisé en ce que** du méthanol, de l'éthanol, du propanol ou du n-butanol est contenu en tant qu'alcool.

5. Procédé selon l'une quelconque des revendications 1 à 4, **caractérisé en ce que** les catalyseurs contiennent au moins un élément du groupe 4, 6, 13 ou 14 de la Classification Périodique des Eléments.

6. Procédé selon la revendication 5, **caractérisé en ce que** les catalyseurs contiennent au moins un composé choisi parmi γ-Al₂O₃ ou TiO₂.

7. Procédé selon l'une quelconque des revendications 1 à 6, **caractérisé en ce que** la réaction est réalisée sur des catalyseurs en suspension en phase liquide ou sur des catalyseurs fixes en phase gazeuse.

8. Procédé selon l'une quelconque des revendications 1 à 7, **caractérisé en ce que** le mélange réactionnel contient 15% à 60% en poids de 1,4-butanediol, 15% à 50% en poids d'un autre ou de plusieurs autres alcools, jusqu'à 30% en poids de γ-butyrolactone et/ou de diester d'acide succinique, cette proportion représentant au maximum la moitié de la proportion de butanediol, et jusqu'à 30% en poids de THF, et le mélange contenant moins de 2% en poids d'eau par rapport à la somme de tous les autres composants formant 100% en poids.

9. Procédé selon l'une quelconque des revendications 1 à 8, **caractérisé en ce que** l'on réalise la réaction en phase gazeuse.

10. Procédé selon l'une quelconque des revendications 1 à 9, **caractérisé en ce que** l'on réalise la réaction à une température de 50°C à 300°C et sous une pression de 0,5 bar à 80 bars.
